(19) Europäisches Patentamt
European Patent Office
Office européen des brevets

(11) Publication number: **0 046 409**
**A1**

(12) # EUROPEAN PATENT APPLICATION

(21) Application number: **81303770.2**

(22) Date of filing: **19.08.81**

(51) Int. Cl.³: **A 61 K 31/375**, C 07 F 1/08, C 07 C 51/41, C 07 C 59/105, A 01 N 43/08

(30) Priority: **20.08.80 AU 5138/80**

(43) Date of publication of application: **24.02.82 Bulletin 82/8**

(84) Designated Contracting States: **AT BE CH DE FR GB IT LI LU NL SE**

(71) Applicant: **Walliczek, Erwin Günther, 13 Biarritz Avenue, Beaumaris Victoria 3193 (AU)**

(72) Inventor: **Walliczek, Erwin Günther, 13 Biarritz Avenue, Beaumaris Victoria 3193 (AU)**

(74) Representative: **Holdcroft, James Gerald et al, Graham Watt & Co. Riverhead, Sevenoaks Kent TN13 2BN (GB)**

(54) **A process for the preparation of a solution of a cuprous complex; solutions of the cuprous complexes for use in the therapeutic treatment of the human or animal body; and a method of therapeutic treatment of a plant.**

(57) A solution of a cuprous complex is prepared by a process in which a cupric halide is dissolved in a solvent consisting of one or more of ethyl alcohol or a liquid primary alcohol containing 3 or more carbon atoms, polypropylene glycol, propylene glycol, polyethylene glycol, ethylene glycol or other liquid glycol, glycerol or other liquid triol, an aliphatic betaine and a sulphobetaine, the concentration of Cu being not less than 0.01% w/v; ascorbic acid not less than 90% of the weight of the Cu present or the equivalent weight of a non-toxic ascorbate, is added and dissolved under an inert atmosphere; and the pH of this solution, if less than 4, is adjusted to 4 to 6 by the addition of an alkali.

The solutions of the cuprous complexes are useful in the topical therapeutic treatment of the human or animal body suffering from any of fungal, imflammatory or viral complaints, arthritis, rheumatism, warts, herpes or carcinoma; papiloma; and also in the topical treatment of plants affected with fungal disorders.

EP 0 046 409 A1

# A PROCESS FOR THE PREPARATION OF A SOLUTION OF A CUPROUS COMPLEX; SOLUTIONS OF THE CUPROUS COMPLEXES FOR USE IN THE THERAPEUTIC TREATMENT OF THE HUMAN OR ANIMAL BODY; AND A METHOD OF THERAPEUTIC TREATMENT OF A PLANT

This invention relates to the preparation of a solution of a cuprous complex and it refers more particularly to one wherein a cupric halide is dissolved in a solvent consisting of one or more of ethyl alcohol or a liquid primary alcohol containing 3 or more carbon atoms, polypropylene glycol, propylene glycol, polyethylene glycol, ethylene glycol or other liquid glycol, glycerol or other liquid triol, an aliphatic betaine e.g. having a pH between 3.5 and 6.0, and a sulpho-betaine e.g. having a pH between 3.5 and 6.0, the concentration of Cu being not less than 0.01% w/v of said solution; ascorbic acid not less than 90% of the weight of the Cu present or the equivalent weight of a non-toxic ascorbate, is added and dissolved under an inert atmosphere; and the pH of this solution, if less than 4, is adjusted to 4 to 6 by the addition of an alkali.

The aliphatic betaines used in embodiments of this invention conform to formula

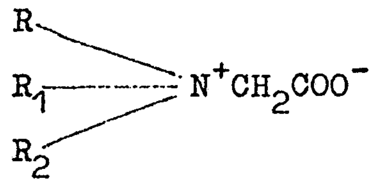

wherein R is an aliphatic radical containing not more than 18 carbon atoms, and optionally containing an amide linkage; $R_1$ and $R_2$, which may be the same or different, are alkyl groups containing not more than 3 carbon atoms and are optionally substituted by hydroxyl.

If the pH of such a compound, as bought commercially, is above 6.0, it may be adjusted to 3.5 to 6.0 by the addition of a hydrohalic acid, before use in the above process.

The sulpho-betaines used in embodiments of this invention conform to formula

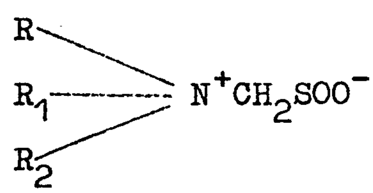

R, $R_1$ and $R_2$ being as defined above. As in the case of the aliphatic betaines, the pH of the commercial product, if above 6.0, is adjusted to 3.5 to 6.0 by the addition of a hydrohalic acid, before use in the above process.

The preferred cupric halide is $CuCl_2.2H_2O$.

The preferred solvents are propylene glycol, and aliphatic betaines of pH between 3.5 and 6.0. Both of these have documented non-toxic properties.

The preferred aliphatic betaines are those in which R is a predominantly alkyl radical having from 12 to 14 carbon atoms, and $R_1$ and $R_2$ are both methyl. These are sold commercially as Empigen BB. If the pH is above 6.0, it is preferably adjusted to 3.5 to 6.0 by strong hydrochloric acid. The preferred pH within the range of 3.5 to 6.0, is 4.

The preferred concentration of Cu in stock solutions which may be diluted for use with a pharmaceutically acceptable diluent, depends on the composition of the solvent. When the solvent is a glycol or glycerol, the preferred concentration of Cu is 10% w/v. When the solvent is an aliphatic betaine of pH 3.5 to 6.0 or a sulpho-betaine of pH 3.5 to 6.0, the preferred concentration of Cu is 5% w/v.

The preferred proportion of ascorbic acid is 100% of the weight of the Cu present. The non-toxic ascorbates include but are not restricted to the ascorbates of alkali metals, alkaline earth metals, aluminium and silicon.

The preferred non-toxic ascorbate is sodium ascorbate, and its preferred proportion is 112.5% of the weight of the Cu present.

The preferred inert atmosphere is nitrogen.

The alkali preferred for adjusting the pH of the

solution to 4 to 6 is 10% w/v sodium hydroxide solution.

While the cupric halide and ascorbic acid or non-toxic ascorbate can sometimes be dissolved in the solvent at ambient temperature, particularly if the solvent is a glycol or triol, heating to a temperature above 60°C is more convenient and sometimes necessary. Some of the aliphatic betaine solutions of the cuprous complex set to a solid at ambient temperature, but can be re-melted at temperatures in the vicinity of 32°C. The preferred temperature range for dissolving the cupric halide is 60°C to 80°C.

The cuprous complex produced by the above process is believed to be a cuprous halo ascorbate. The solutions of the cuprous complex prepared as above have strong antifungal properties when applied topically on humans, animals or plants. The preferred concentration of Cu for topical application to humans or animals afflicted with ringworm or other disability caused by a fungus, is 2 to 3% w/v. The preferred concentration of Cu for topical application to a plant afflicted with a disability caused by a fungus, is 0.5 to 1% w/v. It has also been found that the solution of the cuprous complex prepared by the process above is very effective in the therapeutic treatment of a human or animal suffering from rheumatism, arthritis, or other inflammation; or papiloma, other warts,

herpes, carcinoma or other viral disorder.

The preferred concentration of Cu for topical application for the therapeutic treatment of a human or animal suffering from rheumatism, arthritis or other inflammation, is 2 to 3% w/v.

The preferred concentration of Cu for topical application for the therapeutic treatment of a human or animal suffering from papiloma, other warts, herpes, carcinoma or other viral disorder, is 4 to 5% w/v.

As mentioned above, it is often convenient to prepare a stock solution having 10% w/v or 5% w/v of Cu, and diluting this for use. The preferred pharmaceutically acceptable diluent is propylene glycol.

It is also noted here that solutions of the cuprous complex containing an aliphatic betaine or a sulpho-betaine, are also anti-bacterial. If, therefore, the human or animal to be treated for any of the above disabilities has an associated bacterial infection, it is preferred that the solution of the cuprous complex be one that contains an aliphatic betaine or a sulpho-betaine. This could be a stock solution prepared from a primary alcohol, a glycol or a triol, but subsequently diluted with an aliphatic betaine or a sulpho-betaine.

The following non-limitative Examples illustrate the invention.

EXAMPLE 1

To 1 litre of propylene glycol add 268 g $CuCl_2.2H_2O$. Commence agitation and heat to about 70°C. When the $CuCl_2.2H_2O$ has dissolved, add 100 g ascorbic acid or 112.5 g sodium ascorbate. Without delay, cover the mixture with an atmosphere of nitrogen. After the ascorbic acid or sodium ascorbate has dissolved, propylene glycol may be added to reduce the concentration of Cu to a preferred value for treatment.

Transfer the solution, diluted or not, to ampoules and seal. This product has no anti-bacterial properties.

EXAMPLE 2

Check the pH of 1 litre of an aliphatic betaine sold commercially as Empigen BB. If the pH is above 6.0, add strong hydrochloric acid with agitation until the pH is reduced to 4. With the agitation continued, add 134 g $CuCl_2.2H_2O$ and heat to about 70°C.

When this has dissolved, add 50 g ascorbic acid or 56.25 g sodium ascorbate. Without delay, cover the mixture with an atmosphere of nitrogen. After the ascorbic acid or sodium ascorbate has dissolved, add 10% w/v sodium hydroxide solution until the pH is 5.

Propylene glycol may be added to reduce the concentration of Cu to a preferred value for treatment.

Transfer the solution, diluted or not, to ampoules and seal. This product has anti-bacterial properties.

ANTI-FUNGAL STUDIES

Field tests have shown that a concentration of 2-3% copper applied once daily will clear-up the skin disorder between 2-4 weeks. It is beneficial to apply the medication after a bath has been taken.

ANTI-INFLAMMATION STUDIES

Field tests have shown that a concentration of 2-3% copper applied once daily will diminish rheumatic pain and associated swellings after 3-5 days. Similar results have been obtained on haemmoroids. It is beneficial to apply the medication after a bath has been taken.

ANTI-VIRAL STUDIES

Field tests have shown that a concentration of 4-5% copper applied once daily will completely remove warts, venerial warts, papiloma and dermatose fibroma between 4-6 weeks.

Herpes simplex formation is arrested after a 3-4 day interval. If possible, it is beneficial to apply the medication after a bath has been taken.

CLAIMS: (Except for Austria)

1. A process for the preparation of a solution of a cuprous complex characterised in that (a) a cupric halide is dissolved in a solvent consisting of one or more of ethyl alcohol or a liquid primary alcohol containing 3 or more carbon atoms, polypropylene glycol, propylene glycol, polyethylene glycol, ethylene glycol or other liquid glycol, glycerol or other liquid triol, an aliphatic betaine and a sulpho-betaine the concentration of Cu being not less than 0.01% w/v of said solution; (b) ascorbic acid not less than 90% of the weight of the Cu present or the equivalent weight of a non-toxic ascorbate, is added and dissolved under an inert atmosphere; and (c) the pH of this solution, if less than 4, is adjusted to 4 to 6 by the addition of an alkali.

2. A process as claimed in Claim 1 wherein the cupric halide is dissolved at a temperature between 60°C and 80°C.

3. A process as claimed in Claim 1 or 2 wherein the cupric halide is $CuCl_2.2H_2O$, the non-toxic ascorbate is sodium ascorbate, the weight of ascorbic acid is 100% of the weight of the Cu present or the weight of sodium ascorbate is 112.5% of the weight of the Cu present, and the inert atmosphere is nitrogen.

4. A process as claimed in any preceding

claim wherein the solvent is an aliphatic betaine having a pH between 3.5 and 6.0, a sulpho-betaine having a pH between 3.5 and 6.0 or a mixture of the two.

5. A process as claimed in any preceding claim, wherein the solution is additionally diluted with a pharmaceutically acceptable diluent.

6. A solution of a cuprous complex when produced by a process as claimed in any preceding claim, characterised in that the solution is for use in the topical therapeutic treatment of the human or animal body suffering from any of, fungal, inflammatory or viral complaints; arthritis, rheumatism, warts, herpes or carcinoma; papiloma.

7. A solution according to claim 6, wherein an aliphatic betaine or a sulpho-betaine is present, and characterised in that the solution is for use in the topical therapeutic treatment of the human or animal body also suffering from a bacterial infection associated with any of the said fungal, inflammatory or viral complaints; arthritis, rheumatism, warts, herpes or carcinoma; papiloma.

8. A method of therapeutic treatment of a plant afflicted with a disability caused by a fungus, characterised by topical application of a solution produced by a process as claimed in any one of Claims 1 to 5.

CLAIMS: (for Austria)

1. A process for the preparation of a solution of a cuprous complex characterised in that (a) a cupric halide is dissolved in a solvent consisting of one or more of ethyl alcohol or a liquid primary alcohol containing 3 or more carbon atoms, polypropylene glycol, propylene glycol, polyethylene glycol, ethylene glycol or other liquid glycol, glycerol or other liquid triol, an aliphatic betaine and a sulpho-betaine the concentration of Cu being not less than 0.01% w/v of said solution; (b) ascorbic acid not less than 90% of the weight of the Cu present or the equivalent weight of a non-toxic ascorbate, is added and dissolved under an inert atmosphere; and (c) the pH of this solution, if less than 4, is adjusted to 4 to 6 by the addition of an alkali.

2. A process as claimed in Claim 1 wherein the cupric halide is dissolved at a temperature between 60°C and 80°C.

3. A process as claimed in Claim 1 or 2 wherein the cupric halide is $CuCl_2.2H_2O$, the non-toxic ascorbate is sodium ascorbate, the weight of ascorbic acid is 100% of the weight of the Cu present or the weight of sodium ascorbate is 112.5% of the weight of the Cu present, and the inert atmosphere is nitrogen.

4. A process as claimed in any preceding

claim wherein the solvent is an aliphatic betaine having a pH between 3.5 and 6.0, a sulpho-betaine having a pH between 3.5 and 6.0 or a mixture of the two.

5. A process as claimed in any preceding claim, wherein the solution is additionally diluted with a pharmaceutically acceptable diluent.

6. A method of therapeutic treatment of a plant afflicted with a disability caused by a fungus, characterised by topical application of a solution produced by a process as claimed in any preceding claim.

European Patent Office

# EUROPEAN SEARCH REPORT

0046409

Application number

EP 81 30 3770

| Category | DOCUMENTS CONSIDERED TO BE RELEVANT<br>Citation of document with indication, where appropriate, of relevant passages | Relevant to claim |
|---|---|---|
| | CHEMICAL ABSTRACTS, vol. 73, no. 19, 9th November 1970, page 25, no. 94729b<br>Columbus, Ohio, U.S.A.<br>A. HATANO et al.: "Behavior of copper ion with ascorbic acid"<br>& J. VITAMINOL. (KYOTO) 1970, 16(2) 99-102<br>* Abstract *<br>-- | 1-3 |
| | CHEMICAL ABSTRACTS, vol. 72, no. 11, 16th March 1970, page 26, no. 51083y<br>Columbus, Ohio, U.S.A.<br>A. HATANO et al.: "Ascorbic acid and its complexes. V. Behavior of copper ion with ascorbic acid"<br>& BITAMIN 1969, 40(6), 416-419<br>* Abstract *<br>-- | 1-3 |
| A | FR - A - 2 377 393 (MARSTRAND EVEN)<br>* Page 4, claims 1-7 *<br>---- | 1-8 |

CLASSIFICATION OF THE APPLICATION (Int. Cl.3)

A 61 K 31/375
C 07 F 1/08
C 07 C 51/41
59/105
A 01 N 43/08

TECHNICAL FIELDS SEARCHED (Int. Cl.3)

A 61 K 31/375
C 07 F 1/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 20-11-1981 | SUTER |